# EUROPÄISCHE PATENTANMELDUNG

(11) **EP 0 879 592 A2**
(43) Veröffentlichungstag der Anmeldung: **25.11.1998**
(21) Anmeldenummer: 98106471.0
(22) Anmeldetag: 08.04.1998
(51) Int. Cl.: A61K 7/42, A61K 7/48

(54) **Sonnenschutzmittel**

(30) Priorität: 17.04.1997 DE 19716070
(71) Anmelder: Henkel Kommanditgesellschaft auf Aktien, 40589 Düsseldorf-Holthausen (DE)
(72) Erfinder: Wachter, Rolf, Diplom-Chemiker, 40595 Düsseldorf (DE); Ansmann, Achim, Diplom-Chemiker, 40699 Erkrath (DE); Kühne, Sabine, 42781 Haan (DE)

(57) **Zusammenfassung**

Es werden neue Sonnenschutzmittel vorgeschlagen, enthaltend Ölkörper, Emulgatoren, Chitosane und UV-Lichtschutzfilter. Die Mittel zeichnen sich durch hohe Wasserresistenz, Phasenstabilität sowie besondere hautkosmetische Verträglichkeit aus.

## Beschreibung

### Gebiet der Erfindung

Die Erfindung betrifft Sonnenschutzmittel, enthaltend Ölkörper, Emulgatoren, Chitosane und UV-Lichtschutzfilter sowie die Verwendung der Chitosane zur Herstellung von Sonnenschutzmitteln.

### Stand der Technik

Die Pigmentierung normaler Haut führt unter dem Einfluß von Sonnenstrahlung zur Bildung von Melaninen. Dabei ruft die Bestrahlung mit langwelligem UV-A Licht die Dunkelung der in der Epidermis bereits vorhandenen Melaninkörper hervor, ohne daß schädigende Folgen zu erkennen sind, während die kurzwellige UV-B Strahlung die Bildung neuen Melanins bewirkt. Ehe das schützende Pigment jedoch gebildet werden kann, unterliegt die Haut der Einwirkung der ungefilterten Strahlung, die je nach Expositionsdauer zu Hautrötungen (Erythemen), Hautentzündungen (Sonnenbrand) oder gar Brandblasen führen kann. Die mit derartigen Hautläsionen verbundenen Belastungen des Organismus, beispielsweise im Zusammenhang mit der Ausschüttung von Histaminen, kann zusätzlich zu Kopfschmerzen, Mattigkeit, Fieber, Herz- und Kreislaufstörungen und dergleichen führen. Für den Verbraucher, der sich vor den schädlichen Aspekten der Sonneneinstrahlung schützen will, bietet der Markt eine Vielzahl von Produkten, bei denen es sich ganz überwiegend um Öle und milchige Emulsionen handelt, die neben einigen Pflegestoffen vor allem UV-Lichtschutzfilter enthalten. Übersichten hierzu finden sich beispielsweise von P.Finkel in **Parf.Kosm. 76, 432 (1995)** und S.Schauder in **Parf.Kosm. 76, 490 (1995).**

Dennoch besteht im Markt weiterhin das Bedürfnis nach Produkten mit einem verbesserten Leistungsspektrum. Besonderes Interesse gilt dabei Zubereitungen, die die Einarbeitung von größeren Mengen UV-Lichtschutzfiltern erlauben, ohne daß im Laufe der Lagerung eine Phasentrennung bzw. eine Sedimentation stattfindet. Eine nach der Phaseninversionstemperaturmethode hergestellte Formulierung, wie beispielsweise in der Europäischen Patentanmeldung **EP-A1 0667144** (L'Oreal) beschrieben, neigt bei der Einarbeitung von größeren Mengen Titandioxid sehr rasch zur Ausscheidung des dispergierten Feststoffes. Ein weiteres Problem besteht daß viele UV-Lichtschutzfilter mit den weiteren Bestandteilen der Rezeptur in Wechselwirkung treten können, was zu einer chemische Reaktion und ebenfalls zu einer Abnahme der Lagerbeständigkeit führt. Schließlich wünscht der Verbraucher transparente Formulierungen, die auch gegenüber sehr empfindlicher Haut eine hohe hautkosmetische Verträglichkeit aufweisen und gleichzeitig ausreichend wasserresistent sind, so daß keine Notwendigkeit besteht, die Haut nach jedem Badegang neu einzucremen. Die komplexe Aufgabe der Erfindung hat somit darin bestanden, Sonnenschutzmittel zur Verfügung zu stellen, die sich gleichzeitig durch besondere Wasserresistenz, Phasenstabilität, Lagerbeständigkeit und Verträglichkeit gegenüber empfindlicher Haut auszeichnen.

### Beschreibung der Erfindung

Gegenstand der Erfindung sind Sonnenschutzmittel, enthaltend
(a) Ölkörper,
(b) Emulgatoren,
(c) Chitosane und
(d) UV-Lichtschutzfilter.

Überraschenderweise wurde gefunden, daß Chitosane nicht nur in der Lage sind, Emulsionen so zu stabilisieren, daß feinteilige Formulierungen erhalten werden, die über eine hervorragende Lagerstabilität gerade auch im Temperaturbereich oberhalb von 50°C verfügen, sondern auch außerordentlich wasserbeständig sind. Gleichzeitig werden Zubereitungen mit besonders hoher hautkosmetischer Verträglichkeit erhalten.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv® TN), Dialkylether, Siliconöle und/oder aliphatische bzw. naphthenische Kohlenwasserstoffe in Betracht.

### Emulgatoren

Als Emulgatoren kommen beispielsweise nichtionogene Tenside aus mindestens einer der folgenden Gruppen in Frage:
(b1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(b2) C_{12/18}-Fettsäuremono- und -diester von Anlagerungsprodukten von 1 bis 30 Mol Ethylenoxid an Glycerin;
(b3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(b4) Alkylmono- und -oligoglycoside mit 8 bis 22 Kohlenstoffatomen im Alkylrest und deren ethoxylierte Analoga;
(b5) Anlagerungsprodukte von 15 bis 60 Mol Ethylenoxid an Ricinusöl und/oder gehärtetes Ricinusöl;
(b6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(b7) Anlagerungsprodukte von 2 bis 15 Mol Ethylenoxid an Ricinusöl und/oder gehärtetes Ricinusöl;
(b8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(b9) Trialkylphosphate sowie Mono-, Di- und/oder Tri-PEG-alkylphosphate;
(b10) Wollwachsalkohole;
(b11) Polysiloxan-Polyalkyl-Polyether-Copolymere bzw. entsprechende Derivate;
(b12) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin sowie
(b13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und -oligoglycoside, ihre Herstellung und ihre Verwendung als oberflächenaktive Stoffe sind beispielsweise aus **US 3,839,318, US 3,707,535, US 3,547,828, DE-OS 19 43 689, DE-OS 20 36 472** und **DE-A1 30 01 064** sowie **EP-A 0 077 167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche obenflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

### Chitosane

Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten: 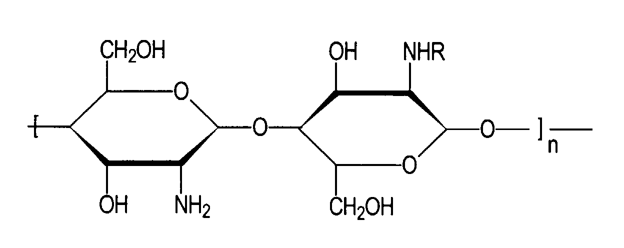 Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt (vgl. **Ullmann's Encyclopedia of Industrial Chemistry, 5th Ed., Vol. A6, Weinheim, Verlag Chemie, 1986, S. 231-332)**. Übersichten zu diesem Thema sind auch beispielsweise von B.Gesslein et al. in **HAPPI 27, 57 (1990)**, O.Skaugrud in **Drug Cosm.Ind. 148, 24 (1991)** und E.Onsoyen et al. in **Seifen-Öle-Fette-Wachse 117, 633 (1991)** erschienen. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Entsprechende Verfahren sind beispielsweise aus **Makromol. Chem. 177, 3589 (1976)** oder der französischen Patentanmeldung **FR-A 2701266** bekannt. Vorzugsweise werden solche Typen eingesetzt, wie sie in den deutschen Patentanmeldungen **DE-A1 4442987** und **DE-A1 19537001** (Henkel) offenbart werden, und die ein durchschnittliches Molekulargewicht von 800.000 bis 1.200.000 Dalton, eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% aufweisen. Neben den Chitosanen als typischen kationischen Biopolymeren kommen im Sinne der Erfindung auch anionisch bzw. nichtionisch derivatisierte Chitosane, wie z.B. Carboxylierungs-, Succinylierungs- oder Alkoxylierungsprodukte in Frage, wie sie beispielsweise in der deutschen Patentschrift **DE-C2 3713099** (L'Oréal) sowie der deutschen Patentanmeldung **DE-A1 19604180** (Henkel) beschrieben werden.

### UV-Lichtschutzfilter

Unter UV-Lichtschutzfiltern sind organische Substanzen zu verstehen, die in der Lage sind, ultraviolette Strahlen zu absorbieren und die aufgenommene Energie in Form längerwelliger Strahlung, z.B. Wärme wieder abzugeben. Eine Übersicht hierzu findet sich beispielsweise in **Parf.Kosm. 74, 485 (1993)**. Typische Beispiele sind 4-Aminobenzoesäure sowie ihre Ester und Derivate (z.B. 2-Ethylhexyl-p-dimethylaminobenzoat oder p-Dimethylaminobenzoesäureoctylester), Methoxyzimtsäure und ihre Derivate (z.B. 4-Methoxyzimtsäure-2-ethylhexylester), Benzophenone (z.B. Oxybenzon, 2-Hydroxy-4-methoxy-benzophenon), Dibenzoylmethane, Salicylatester, 2-Phenylbenzimadozol-5-sulfonsäure, 1-(4-tert.Butylphenyl)-3-(4'-methoxyphenyl)-propan-1,3-dion, 3-(4'-Methyl)benzyliden-bornan-2-on, Methylbenzylidencampher und dergleichen.

Die erfindungsgemäßen Mittel können als Lichtschutzfilter des weiteren auch feindisperse Metalloxide bzw. Salze enthalten. Typische Beispiele sind Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen.

Neben den beiden vorgenannten Gruppen primärer Lichtschutzstoffe können die erfindungsgemäßen Mittel ferner auch sekundäre Lichtschutzmittel vom Typ der Antioxidantien enthalten, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Der Anteil der Lichtschutzmittel an den erfindungsgemäßen Mitteln liegt - bezogen auf den nichtwäßrigen Anteil - üblicherweise bei 10 bis 90, vorzugsweise 25 bis 75 und insbesondere 40 bis 60 Gew.-%. Die erfindungsgemäßen Mittel als solche können 1 bis 95, vorzugsweise 5 bis 80 und insbesondere 10 bis 60 Gew.-% Wasser enthalten. Werden als Lichtschutzmittel organische Verbindungen eingesetzt, können zur Herstellung der Zubereitungen deren co-emulgierenden Eigenschaften mitgenutzt werden.

### Gewerbliche Anwendbarkeit

Die erfindungsgemäßen Mittel zeichnen sich durch eine hohe Wasserresistenz und Phasenstabilität bei besonders vorteilhafter hautkosmetischer Verträglichkeit aus. Eine weiterer Gegenstand der Erfindung betrifft daher die Verwendung der Chitosane als Wasserrepellentien zur Herstellung von Sonnenschutzmitteln. Solche Zubereitungen haben beispielsweise die folgende Zusammensetzung:
(a) 1 bis 30, vorzugsweise 5 bis 20 Gew.-% Ölkörper,
(b) 1 bis 20, vorzugsweise 2 bis 10 Gew.-% Emulgatoren,
(c) 1 bis 5, vorzugsweise 1 bis 2 Gew.-% Chitosane und
(d) 1 bis 20, vorzugsweise 2 bis 10 Gew.-% UV-Lichtschutzfilter,
mit der Maßgabe, daß sich die Mengenangaben mit Wasser zu 100 Gew.-% ergänzen.

Als weitere Inhaltsstoffe können sie in untergeordneten Mengen weitere, mit den anderen Inhaltsstoffen kompatible Aniontenside und/oder Niotenside enthalten. Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkyl-sulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Vorzugsweise werde Alkylethersulfate und/oder Alkyl(ether)phosphate eingesetzt. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Die erfindungsgemäßen Mittel können ferner als weitere Hilfs- und Zusatzstoffe Überfettungsmittel, Stabilisatoren, Wachse, Konsistenzgeber, Verdickungsmittel, Kationpolymere, biogene Wirkstoffe, Konservierungsmittel, Hydrotrope, Solubilisatoren, Farb- und Duftstoffe enthalten.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. ein quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinyl-imidazol-Polymere wie z.B. Luviquat® (BASF), Kondensationsprodukte von Polyglycolen und Aminen, quaternierte Kollagenpolypeptide wie beispielsweise Lauryldimonium hydroxypropyl hydrolyzed collagen (Lamequat®/Grünau), quaternierte Weizenpolypeptide, Polyethylenimin, kationische Siliconpolymere wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide wie z.B. beschrieben in der **FR-A 2252840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese, quaternierte Ammoniumsalz-Polymere wie z.B. Mirapol® A-15, Mirapol® AD-1, Mirapol® AZ-1 der Miranol.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Carnaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Perlglanzwachse** können insbesondere Mono- und Difettsäureester von Polyalkylenglycolen, Partialglyceride oder Ester von Fettalkoholen mit mehrwertigen Carbonsäuren bzw. Hydroxycarbonsäuren verwendet werden. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Tocopherol, Tocopherolacetat, Tocopherolpalmitat, Ascorbinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen.

Zur Verbesserung des Fließverhaltens können ferner **Hydrotrope** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Als **Konservierungsmittel** eignen sich beispielsweise Phenoxyethanol, Formaldehydlösung, Parabene, Pentandiol oder Sorbinsäure. Als **Insekten-Repellentien** kommen N,N-Diethyl-m-touluamid, 1,2-Pentandiol oder Insect repellent 3535 in Frage, als **Selbstbräuner** eignet sich Dihydroxyaceton. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

Die Sonnenschutzemulsionen R1 bis R8 wurden in einem Heißverfahren hergestellt. Dazu wurden die Ölkörper zusammen mit den öllöslichen bzw. öldispergierbaren UV-Lichtschutzfiltern und den Emulgatoren bei 70 bis 80°C in Wasser gleicher Temperatur emulgiert; wasserlösliche bzw. wasserdispergierbare UV-Filter wurden zusammen mit der wäßrigen Phase eingebracht. Die Beurteilung der Stabilität erfolgte nach einer Lagerung von 10 Tagen bei 50 °C. Hierbei bedeuten (+) = keine Phasentrennung/Sedimentation und (-) Phasentrennung/Trübung. Die Wasserresistenz wurde in einem Paneltest untersucht. Hierbei bedeutet (+) bei einmaligem Eincremen und UV-Exposition keine Hautrötung trotz dreimaligem Waschen der Haut und (-) Hautrötung unter den gleichen Bedingungen. Die Rezepturen 1 bis 7 sind erfindungsgemäß, die Rezeptur V1 dient zum Vergleich. Die Ergebnisse sind in Tabelle 1 zusammengefaßt.

**Tabelle 1**

| **O/W-Sonnenschutzemulsionen (Mengenangaben als Gew.-%).** | | | | | | | | |
|---|---|---|---|---|---|---|---|---|
| **Komponente (INCI)** | **1** | **2** | **3** | **4** | **5** | **6** | **7** | **V1** |
| Coco Glycerides | 10,0 | - | 12,0 | 10,0 | - | 17,0 | - | 10,0 |
| Octyl Dodecanol | - | 16,0 | - | - | 18,0 | - | - | - |
| Almond oil | - | - | - | - | - | - | 16,0 | - |
| Cetearyl Glucoside (and) Cetearyl Alcohol (50 : 50) | 4,0 | 4,2 | 4,2 | 2,0 | 4,5 | 3,0 | - | 4,0 |
| Ceteareth-20 | - | - | - | - | - | - | - | - |
| Polylglyceryl-3 Methylglucose Distearate | - | - | - | 2,0 | - | - | - | - |
| Polyglyceryl-2 Dihydroxystearate (and) Lauryl Glucoside (and) Glycerin | - | - | 1,0 | - | - | - | - | - |
| Chitosan | 0,1 | 0,2 | 0,5 | 1,0 | - | - | - | - |
| Succinyliertes Chitosan | - | - | - | - | 0,8 | 0,8 | 0,8 | - |
| Benzophenone-3 | 2,0 | 2,0 | - | 2,0 | - | 2,0 | 2,0 | 2,0 |
| Octyl Methoxycinnamate | 7,5 | 7,5 | 7,5 | 7,5 | - | 3,0 | 7,5 | 7,5 |
| Titandioxid | - | - | 5,0 | - | 5,0 | 4,5 | - | - |
| Zinkoxid | - | - | - | - | 5,0 | 3,5 | - | - |
| Octyl Triazone | - | 6,0 | - | - | - | - | - | - |
| Glycerin | 5,0 | 5,0 | 5,0 | 5,0 | 5,0 | 5,0 | 5,0 | 5,0 |
| Wasser | ad 100 | | | | | | | |
| ***Stabilität*** | + | + | + | + | + | + | + | - |
| ***Wasserresistenz*** | + | + | + | + | + | + | + | - |

## Patentansprüche

1. Sonnenschutzmittel, enthaltend
(a) Ölkörper,
(b) Emulgatoren,
(c) Chitosane und
(d) UV-Lichtschutzfilter.

2. Mittel nach Anspruch 1, **dadurch gekennzeichnet,** daß sie Ölkörper enthalten, die ausgewählt sind aus der Gruppe, die gebildet wird Guerbetalkoholen auf Basis von Fettalkoholen mit 6 bis 18 Kohlenstoffatomen, Estern von linearen C₆-C₂₂-Fettsäuren mit linearen C₆-C₂₂-Fettalkoholen, Estern von verzweigten C₆-C₁₃-Carbonsäuren mit linearen C₆-C₂₂-Fettalkoholen, Estern von linearen C₆-C₂₂-Fettsäuren mit verzweigten Alkoholen, Estern von linearen und/oder verzweigten Fettsäuren mit mehrwertigen Alkoholen und/oder Guerbetalkoholen, Triglyceriden auf Basis C₆-C₁₀-Fettsäuren, flüssigen Mono-/Di-/Triglyceridmischungen auf Basis von C₆-C₁₈-Fettsäuren, Estern von C₆-C₂₂-Fettalkoholen und/oder Guerbetalkoholen mit aromatischen Carbonsäuren, pflanzlichen Ölen, verzweigten primären Alkoholen, substituierten Cyclohexanen, linearen C₆-C₂₂-Fettalkoholcarbonaten, Guerbetcarbonaten, Estern der Benzoesäure mit linearen und/oder verzweigten C₆-C₂₂-Alkoholen, Dialkylethern, Siliconölen und/oder aliphatischen bzw. naphthenischen Kohlenwasserstoffen.

3. Mittel nach den Ansprüchen 1 und 2, **dadurch gekennzeichnet,** daß sie Emulgatoren enthalten, die ausgewählt sind aus der Gruppe, die gebildet wird von
(b1) Anlagerungsprodukten von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(b2) C_{12/18}-Fettsäuremono- und -diestern von Anlagerungsprodukten von 1 bis 30 Mol Ethylenoxid an Glycerin;
(b3) Glycerinmono- und -diestern und Sorbitanmono- und -diestern von gesättigten und ungesättigten Fettsäuren mit 6 bis 22 Kohlenstoffatomen und deren Ethylenoxidanlagerungsprodukten;
(b4) Alkylmono- und -oligoglycosiden mit 8 bis 22 Kohlenstoffatomen im Alkylrest und deren ethoxylierten Analoga;
(b5) Anlagerungsprodukten von 15 bis 60 Mol Ethylenoxid an Ricinusöl und/oder gehärtetes Ricinusöl;
(b6) Polyol- und insbesondere Polyglycerinestern;
(b7) Anlagerungsprodukten von 2 bis 15 Mol Ethylenoxid an Ricinusöl und/oder gehärtetes Ricinusöl;
(b8) Partialestern auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkoholen, Alkylglucosiden sowie Polyglucosiden;
(b9) Trialkylphosphaten sowie Mono-, Di- und/oder Tri-PEG-alkylphosphaten;
(b10) Wollwachsalkoholen;
(b11) Polysiloxan-Polyalkyl-Polyether-Copolymeren bzw. entsprechende Derivaten;
(b12) Mischestern aus Pentaerythrit, Fettsäuren, Citronensäure und Fettalkohol und/oder Mischester von Fettsäuren mit 6 bis 22 Kohlenstoffatomen, Methylglucose und Polyolen;
(b13) Polyalkylenglycolen;
(b14) Betainen;
(b15) Esterquats.

4. Mittel nach den Ansprüchen 1 bis 3, **dadurch gekennzeichnet,** daß sie UV-Lichtschutzfilter enthalten, die ausgewählt sind aus der Gruppe, die gebildet wird von 4-Aminobenzoesäure sowie ihren Estern und Derivaten, Methoxyzimtsäure und ihren Derivaten, Benzophenonen, Dibenzoylmethanen, Salicylatestern, 2-Phenylbenzimadozol-5-sulfonsäure, 1-(4-tert.Butylphenyl)-3-(4'-methoxyphenyl)-propan-1,3-dion, 3-(4'-Methyl)benzyliden-bornan-2-on und Methylbenzylidencampher.

5. Mittel nach den Ansprüchen 1 bis 4, **dadurch gekennzeichnet,** daß sie als UV-Lichtschutzfilter feindisperse Metalloxide bzw. Salze enthalten, die ausgewählt sind aus der Gruppe, die gebildet wird von Titantoxid, Zinkoxid, Eisenoxid, Aluminiumoxid, Ceroxid, Zirkoniumoxid, Silicate (Talk) und Bariumsulfat.

6. Mittel nach den Ansprüchen 1 bis 5, **dadurch gekennzeichnet,** daß sie als UV-Lichtschutzmittel Antioxidantien enthalten, die ausgewählt sind aus der Gruppe, die gebildet wird von Superoxid-Dismutase, Tocopherolen (Vitamin E) und Ascorbinsäure (Vitamin C).

7. Verwendung von kationischen Biopolymeren als Wasserrepellentien zur Herstellung von Sonnenschutzmitteln.
